# EUROPÄISCHE PATENTANMELDUNG

(11) **EP 0 582 933 A1**
(43) Veröffentlichungstag der Anmeldung: **16.02.1994**
(21) Anmeldenummer: 93112390.5
(22) Anmeldetag: 03.08.1993
(51) Int. Cl.: A61K 37/02, A61K 9/08, A61M 5/142

(54) **Therapeutisches System zur parenteralen Verabreichung von hämatopoetischen Wachstumsfaktoren**

(30) Priorität: 11.08.1992 CH 2519/92
(71) Anmelder: F. HOFFMANN-LA ROCHE AG, CH-4002 Basel (CH)
(72) Erfinder: Cirelli, Giorgio, CH-4313 Möhlin (CH); Steffen, Hans, CH-4410 Liestal (CH)
(74) Vertreter: Buntz, Gerhard

(57) **Zusammenfassung**

Durch die Kombination einer geeignet formulierten Injektionslösung von hämatopoetischen Wachstumsfaktoren mit einem selbsttätigen, von dem Patienten getragenen Injektionsgerät zu einem therapeutischen System wird die Möglichkeit zur Selbstmedikation von bisher nur im stationären Aufenthalt verabreichbaren Wirkstoffen geschaffen.

## Beschreibung

Die Erfindung betrifft ein therapeutisches System zur Verabreichung von hämatopoetischen Wachstumsfaktoren, speziell von humanem Erythropoetin (EPO). Die Erfindung betrifft ausserdem ein tragbares Injektionsgerät mit einer Haltevorrichtung zur Befestigung des Gerätes am Körper des Patienten, mit einer Injektionsnadel, einer Pumpeinrichtung zum Entleeren der Wirkstofflösung aus dem Behälter durch die Injektionsnadel und einer Nadelantriebsvorrichtung zum Einschiessen der Nadel in die Haut des Patienten.

Für den Zweck dieser Anmeldung umfasst der Begriff humanes Erythropoetin (EPO) in dieser Anmeldung das natürlich vorkommende, humane EPO Protein, Teile dieses Moleküls sowie sämtliche Polypeptid-Analoge oder Derivate von EPO die einige oder alle Eigenschaften der natürlich vorkommenden Moleküle aufweisen. Zu den Derivaten gehören auch solche, die wiederum von den natürlich vorkommenden Sequenzen leicht abweichen, sei es in der Identität oder lokalen Anordnung von einer oder mehreren Aminosäuren, und auch pegylierte oder glykosylierte Formen des EPO.

EPO stimuliert die Erythropoese und wird bei symptomatischen oder transfusionsbedingten Anämien eingesetzt, welche infolge chronischer Nierenerkrankungen bei hämodialysierten Patienten auftreten.

Proteine werden in der Regel im Magen-Darm-Trakt abgebaut und weisen deshalb nach oraler Verabreichung eine sehr geringe Resorption auf. Die Zuführung solcher Wirkstoffe erfolgt daher üblicherweise durch verschiedene parenterale Darreichungsarten wie Injektion, Infusion u.a.m., welche nicht nur mit Injektionsschmerzen verbunden sind, sondern zusätzlich noch die Selbstapplikation weitgehend verhindern. Für die Verabreichung von EPO werden heute lediglich konventionelle, intravenöse oder subkutane Injektionen mit herkömmlichen Spritzen bzw. Infusionsbestecken angewendet. Diese Applikationsarten bringen den schwerwiegenden Nachteil mit sich, dass der Patient während der Therapiedauer in der Regel auf die Hilfe medizinisch geschulten Personals angewiesen ist, sei es im Krankenhaus oder zu Hause. Selbstmedikation ist deshalb bis heute weitgehend ausgeschlossen.

Der Erfindung liegt die Aufgabe zugrunde, diesen Nachteil zu beheben, indem eine Möglichkeit zur schmerzfreien Selbstmedikation geschaffen wird.

Erfindungsgemäss wird diese Aufgabe, wie in den Ansprüchen näher gekennzeichnet, gelöst.

Das für die Injektion von EPO verwendete tragbare Injektionsgerät hat den Vorteil, dass das Einschiessen der Injektionsnadel von Patienten lediglich ausgelöst wird, aber sonst automatisch und weitgehend schmerzfrei erfolgt und dass das Gerät danach die Injektion, ohne weitere vom Patienten durchzuführende Massnahmen, selbsttätig vornimmt.

Im folgenden wird ein bevorzugtes Ausführungsbeispiel der Erfindung beschrieben.

Für das vorliegende therapeutische System geeignete Injektionsgeräte sind aus EP-A 272 530 bekannt. Sie enthalten einen Vorratsbehälter für die Wirkstofflösung und eine mit dem Vorratsbehälter verbundene Injektionsnadel. Die Injektionsnadel ist an einem Nadelträger befestigt, der Teil einer Nadelantriebsvorrichtung ist. Diese Nadelantriebsvorrichtung besitzt eine auf den Nadelträger wirkende Antriebsfeder zum Einschiessen der Nadel in die Haut des Patienten, sowie eine Halteeinrichtung zum Festhalten des Nadelträgers bei gespannter Feder. Die Halteeinrichtung enthält eine Sicherungseinrichtung zur Vermeidung einer unbeabsichtigten Betätigung.

Dem Vorratsbehälter ist eine Pumpeinrichtung zugeordnet, die der Entleerung der Wirkstofflösung durch die Nadel in die Haut des Patienten dient. Zur Flussregulierung der Wirkstofflösung und, damit verbunden, der Abstimmung von Injektionsmenge und -dauer sind der Pumpeinrichtung Drosselelemente zugeordnet. Zur Befestigung des Systems am Patienten dient eine Klebeschicht.

Für den Einsatz von hämatopoetischen Wirkstoffen in dem beschriebenen therapeutischen System eignen sich an sich bekannte pharmazeutische Formulierungen, die neben dem Wirkstoff geeignete Lösungsmittel, Hilfsstoffe und oder Trägerstoffe enthalten.

Die folgende Formulierung von EPO ist ein Beispiel einer Wirkstofflösung, welche mit dem beschriebenen System verabreicht werden kann:

| | |
|---|---|
| Epoetin alfa | 4'000 Einheiten |
| Human Serum Albumin | 2.5 mg |
| Zitronensäure | 0.06 mg |
| Natrium citrat | 5.8 mg |
| Natrium chlorid | 5.8 mg |
| Wasser zu Injektionszwecken | ad 1.0 ml |

Der pH der Lösung beträgt 7.0. Die Einstichtiefe kann in diesem Falle z.B. 2 mm betragen.

## Patentansprüche

1. Therapeutisches System zur parenteralen Verabreichung von hämatopoetischen Wachstumsfaktoren gekennzeichnet durch eine Injektionslösung mit diesen Faktoren als Wirkstoff im Vorratsbehälter eines am Körper tragbaren Injektionsgerätes mit einer Injektionsnadel, einer Pumpeinrichtung zum Entleeren des Behälters durch die Injektionsnadel, einer Nadelantriebsvorrichtung zum Einschiessen der Nadel in die Haut des Patienten und einer Haltevorrichtung zur Befestigung des Systems am Körper des Patienten.

2. Therapeutisches System nach Anspruch 1, dadurch gekennzeichnet, dass der hämatopoetische Wachstumsfaktor humanes Erythropoetin ist.

3. Therapeutisches System nach einem der Ansprüche 1-2, dadurch gekennzeichnet, dass die Injektionslösung eine pharmazeutische Formulierung ist, welche wirksame Mengen des Wirkstoffes mit den in diesem Zusammenhang als geeignet bekannten Lösungsmitteln, Hilfsstoffen und/oder Trägerstoffen enthält, die für die Therapie mit den Wirkstoffen nützlich sind.

4. Therapeutisches System nach einem der Ansprüche 1-3, dadurch gekennzeichnet, dass die Injektionslösung eine pharmazeutische Formulierung ist, welche zusätzlich zum Wirkstoff wenigstens eine pharmazeutisch annehmbare oberflächenaktive Verbindung, ein Saccharid, ein Protein oder ein Makromolekül enthält.

5. Therapeutisches System nach Anspruch 4, gekennzeichnet durch wenigstens einen der folgenden Bestandteile: Aminosäure, Disulfidreduzierende Mittel, Antioxidantien, Komplexbildner, Lösungsmittel, Lösungsvermittler, isotonisierende Hilfsstoffe, Trägerstoffe, basische und saure Hilfsstoffe, Salze und Salzbildner, schmerzlinderndes Mittel, Konservierungsmittel und Puffersubstanzen.

6. Tragbares Injektionsgerät mit einer Haltevorrichtung zur Befestigung des Gerätes am Körper des Patienten, mit einer Injektionsnadel, einer Pumpeinrichtung zum Entleeren des Behälters durch die Injektionsnadel und einer Nadelantriebsvorrichtung zum Einschiessen der Nadel in die Haut des Patienten, dadurch gekennzeichnet, dass der Vorratsbehälter eine Injektionslösung mit hämatopoetischen Wachstumsfaktoren als Wirkstoff enthält.

7. Tragbares Injektionsgerät nach Anspruch 6, dadurch gekennzeichnet, dass der hämatopoetische Wachstumsfaktor humanes Erythropoetin ist.
